# EUROPEAN PATENT APPLICATION

(11) **EP 3 576 093 A1**
(43) Date of publication of application: **04.12.2019**
(21) Application number: 19176772.2
(22) Date of filing: 27.05.2019
(51) Int. Cl.: G16H 10/20

(54) **METHOD AND SYSTEM FOR CLINICAL EFFECTIVENESS EVALUATION OF ARTIFICIAL INTELLIGENCE BASED MEDICAL DEVICE**

(30) Priority: 30.05.2018 KR 20180061698
(71) Applicant: JLK Inspection Inc., Chungcheongbuk-do 28116 (KR)
(72) Inventor: KIM, Won Tae, 16701 Gyeonggi-do (KR); KANG, Shin Uk, 06276 Seoul (KR); LEE, Myung Jae, 06090 Seoul (KR); KIM, Dong Min, 06276 Seoul (KR); JANG, Jin Seong, 05720 Seoul (KR); LEE, Hangbin, 08746 Seoul (KR)
(74) Representative: Brann AB

(57) **Abstract**

There is disclosed a method and system for clinical effectiveness evaluation of artificial intelligence based medical devices. The clinical effectiveness evaluation system includes a diagnosis result receiving unit receiving diagnosis results for arbitrary clinical data from a plurality of specialists and the artificial intelligence based medical device; a correspondence degree calculation unit calculating a degree of correspondence between the received diagnosis results; a statistical value derivation unit deriving a predetermined statistical value on the basis of the calculated degree of correspondence; and a comparison test unit performing a comparison test on the artificial intelligence based medical device using the derived statistical value.

## Description

### CROSS REFERENCE TO RELATED APPLICATION

The present application claims priority to Korean Patent Application Nos. 10-2018-0061698, filed May 30, 2018, the entire contents of which is incorporated herein for all purposes by this reference.

### BACKGROUND OF THE INVENTION

### Field of the Invention

The present disclosure relates to a method and system for clinical effectiveness evaluation of artificial intelligence (AI) based medical devices.

### Description of the Related Art

Recently, demands for artificial intelligence based medical devices have increased. For example, in the case of a cerebral infarction diagnostic medical device based on artificial intelligence, a cerebral infarction pattern is learned in an artificial neural network using medical big data related to cerebral infarction, and type classification of cerebral infarction can be performed on the basis of the learned data.

In order to clinically use an artificial intelligence based medical device, it is necessary to verify the clinical effectiveness for the diagnosis result thereof. As a technique for verifying the clinical effectiveness of an artificial intelligence based medical device in the related art, there is known a technique for collecting diagnosis correspondences among specialists from a research record preceding the clinical test to calculate an average diagnosis correspondence and comparing the average diagnostic correspondence with the diagnostic correspondence between the specialist participating in clinical test and the artificial intelligent based medical device (refer to Korean Patent No. 10-1811028).

However, since the experimental environment and subjects are different for each study, simple comparison or simple average values may cause problems in the reliability of the evaluation results. Specifically, when the data used in clinical test and the experimental environment are somewhat different from the preceding studies, there is a risk that distorted results may be obtained. In addition, there is a limitation that effectiveness evaluation is impossible when there is no precedent study in which correspondence is recorded.

### SUMMARY OF THE INVENTION

Accordingly, an object of the present disclosure is to provide a method and system for clinical effectiveness evaluation of an artificial intelligence based medical device, which can verify the clinical effectiveness for a diagnosis result of the artificial intelligence based medical device.

It is another object of the present disclosure to provide a method and system for clinical effectiveness evaluation of an artificial intelligence based medical device, which can enhance the reliability of diagnosis results generated from the artificial intelligence based medical device.

The technical objects to be achieved by the present disclosure are not limited to the above-mentioned technical subjects, and other technical subjects which are not mentioned are to be clearly understood from the following description to those skilled in the art.

According to an aspect of the present disclosure, a system for clinical effectiveness evaluation of an artificial intelligence based medical device is provided. The system includes a diagnosis result receiving unit receiving diagnosis results for arbitrary clinical data from a plurality of specialists and the artificial intelligence based medical device (also referred to as "AI medical device"); a correspondence degree calculation unit calculating a degree of correspondence between the received diagnosis results; a statistical value derivation unit deriving a predetermined statistical value on the basis of the calculated degree of correspondence; and a comparison test unit performing a comparison test on the artificial intelligence based medical device using the derived statistical value.

According to another aspect of the present disclosure, a method for clinical effectiveness evaluation of an artificial intelligence based medical device is provided. The method includes receiving unit receiving diagnosis results for arbitrary clinical data from a plurality of specialists and the artificial intelligence based medical device (also referred to as "AI medical device"); calculating a degree of correspondence between the received diagnosis results; deriving a predetermined statistical value on the basis of the calculated degree of correspondence; and performing a comparison test on the artificial intelligence based medical device using the derived statistical value.

According to another aspect of the present disclosure, a software or computer readable medium having executable instructions is provided in order to perform the method for clinical effectiveness evaluation of the artificial intelligence based medical device according to the present disclosure.

The features briefly summarized above for this disclosure are only exemplary aspects of the detailed description of the disclosure which follow, and are not intended to limit the scope of the disclosure.

According to the present disclosure, it is possible to provide a method and system for clinical effectiveness evaluation of an artificial intelligence based medical device, which can verify the clinical effectiveness for a diagnosis result of the artificial intelligence based medical device.

In addition, according to the present disclosure, a method and system for clinical effectiveness evaluation of an artificial intelligence based medical device, which can enhance the reliability of diagnosis results generated from the artificial intelligence based medical device, can be provided.

The technical objects to be achieved by the present disclosure are not limited to the above-mentioned technical subjects, and other technical subjects which are not mentioned will be clearly understood from the following description to those skilled in the art.

### BRIEF DESCRIPTION OF THE DRAWINGS

The above and other objects, features, and other advantages of the present invention will be more clearly understood from the following detailed description when taken in conjunction with the accompanying drawings, in which:
FIG. 1 is a diagram illustrating a system for clinical effectiveness evaluation of an artificial intelligence based medical device according to the present disclosure;
FIG. 2 is a diagram illustrating a method for clinical effectiveness evaluation of an artificial intelligence based medical device according to the present disclosure; and
FIG. 3 is a block diagram illustrating a computing system that performs a method for clinical effectiveness evaluation of an artificial intelligence based medical device according to an embodiment of the present disclosure.

### DETAILED DESCRIPTION OF THE INVENTION

Hereinafter, embodiments of the present disclosure will be described in detail with reference to the accompanying drawings so that those skilled in the art can easily carry out the present disclosure. However, the present disclosure may be embodied in many different forms and is not limited to the embodiments described herein.

In the following description of the embodiments of the present disclosure, a detailed description of known functions and configurations incorporated herein will be omitted when it may make the subject matter of the present disclosure rather unclear. Parts not related to the description of the present disclosure in the drawings are omitted, and like parts are denoted by similar reference numerals.

In the present disclosure, when a component is referred to as being "connected" or "coupled" to another component, the component may be directly connected or coupled to the another component, but it is to be understood that there may be other components in between. Also, when a component is referred to as "comprising" or "having" another component, it is appreciated that this is not to exclude other elements but to further include any other elements unless explicitly mentioned to the contrary.

In the present disclosure, the terms first, second, etc. are used only for the purpose of distinguishing one component from another, and do not limit the order or importance of the components unless specifically mentioned. Thus, within the scope of this disclosure, a first component in one embodiment may be referred to as a second component in another embodiment, and similarly a second component in one embodiment may be referred to as a second component in another embodiment.

In the present disclosure, components that are distinguished from each other are intended to clearly illustrate each feature and do not necessarily mean that components are separate. That is, a plurality of components may be integrated into one hardware or software unit, or a single component may be distributed into a plurality of hardware or software units. Accordingly, such integrated or distributed embodiments are also included within the scope of the present disclosure, unless otherwise noted.

In the present disclosure, the components described in the various embodiments do not necessarily mean essential components, and some may be optional components. Accordingly, embodiments consisting of a subset of the components described in one embodiment are also included within the scope of this disclosure. Also, embodiments that include other components in addition to the components described in the various embodiments are also included in the scope of the present disclosure.

Hereinafter, embodiments of the present disclosure will be described with reference to the accompanying drawings.

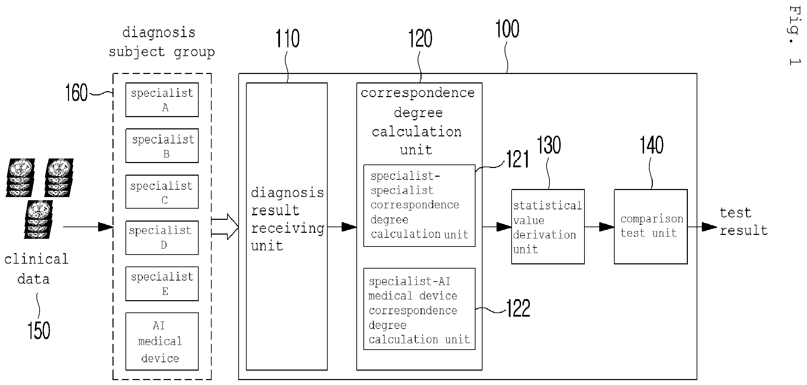

FIG. 1 is a diagram illustrating a system for clinical effectiveness evaluation (hereinafter, referred to as "clinical effectiveness evaluation system") of an artificial intelligence based medical device (hereinafter, referred to as "AI medical device) according to the present disclosure.

The clinical effectiveness evaluation system 100 according to the present disclosure may include a diagnosis result receiving unit 110, a correspondence degree calculation unit 120, a statistical value derivation unit 130, and/or a comparison test unit 140. The clinical effectiveness evaluation system 100 may also be referred to as a clinical effectiveness evaluation device.

Clinical data 150 may be one of the inputs for evaluating the clinical effectiveness of the AI medical device. The diagnostic subject group 160 that performs the diagnosis on the clinical data 150 may include two or more specialists and an AI medical device. The plurality of specialists included in the diagnostic subject group 160 may be two or more, and in the embodiment described below, five specialists ("specialist A", "specialist B", "specialist C", "specialist D", and "specialist E") are included in the diagnostic subject group 160. The AI medical device included in the diagnostic subject group 160 may be the subject of clinical effectiveness evaluation according to the present disclosure. Each of the plurality of specialists and AI medical devices belonging to the diagnostic subject group 160 may perform the diagnosis on the basis of the clinical data 150 and generate diagnosis results. The diagnosis results of "specialist A" to "specialist E" are referred to as a "diagnosis result A" to a "diagnosis result E", respectively. The diagnosis result of the AI medical device is referred to as an "AI diagnosis result".

The clinical effectiveness evaluation system 100 according to the present disclosure may receive the "diagnosis result A" to the "diagnosis result E" and the "AI diagnosis result" through the diagnosis result receiving unit 110.

The correspondence degree calculation unit 120 may calculate the degree of correspondence between diagnosis results on the basis of a plurality of diagnosis results received at the diagnosis result receiving unit 110. The correspondence degree calculation unit 120 may include a specialist-specialist correspondence degree calculation unit 121 and/or a specialist-AI medical device correspondence degree calculation unit 122.

The specialist-specialist correspondence degree calculation unit 121 may calculate the degree of correspondence between diagnosis results of two specialists (hereinafter, referred to as "specialist-specialist degree of correspondence). That is, the specialist-specialist correspondence degree calculation unit 121 may calculate the degree of correspondence between two diagnosis results selected among the "diagnosis result A" to the "diagnosis result E". For example, when the total number of diagnosis results of specialists is 5, 10 combinations are obtained by selecting two diagnosis results among from the 5 diagnosis results. Thus, the specialist-specialist correspondence degree calculation unit 121 may calculate a total of 10 degrees of correspondence.

A specialist-AI medical device correspondence degree calculation unit 122 may calculate the degree of correspondence between the diagnosis result of one specialist and the diagnosis result of the AI medical device, which is the subject of the clinical effectiveness evaluation according to the present disclosure (hereinafter, referred to as "specialist-AI medical device degree of correspondence). That is, the specialist-AI medical device correspondence degree calculation unit 122 may calculate the degree of correspondence between the "AI diagnosis result" and one diagnosis result selected from the "AI diagnosis result" and the "diagnosis result A" to the "diagnosis result E". For example, when there are five diagnosis results made by specialists, the degree of correspondence between the respective diagnosis results and the "AI diagnosis result" may be calculated. Accordingly, in the embodiment described with reference to FIG. 1, the specialist-AI medical device correspondence degree calculation unit 122 may calculate a total of five degrees of correspondence.

Cohen's kappa statistics may be used as a measure of evaluating the degree of correspondence of the diagnosis results between specialists and/or the degree of correspondence of the diagnosis results between the AI medical device and the specialist. In the embodiment shown in FIG. 1, the ten degrees of correspondence calculated by the specialist-specialist correspondence degree calculation unit 121 may be expressed as κ_{0,1} to κ_{0,10}, respectively. Also, the five degrees of correspondence calculated by the specialist-AI medical device correspondence degree calculation unit 122 may be expressed as κ_{1,1} to κ_{1,5}, respectively. Each of the degrees of correspondence κ_{0,1} to κ_{0,10} and κ_{1,1} to κ_{1,5} may be kappa statistics.

The statistical value derivation unit 130 may receive the degree of correspondence calculated in the correspondence degree calculation unit 120 and derive a predetermined statistical value. For example, when the predetermined statistical value is an average, the statistical value derivation unit 130 receives the κ_{0,1} to κ_{0,10} calculated in the specialist-specialist correspondence degree calculation unit 121 and calculates an average of these values (hereinafter, referred to as "specialist-specialist average degree of correspondence"). The statistical value derivation unit 130 receives κ_{1,1} to κ_{1,5} from the specialist-AI medical device correspondence degree calculation unit 122 and calculates an average of these values (hereinafter, referred to as "specialist-AI medical device average degree of correspondence"). The statistical value derivation unit 130 may separately include a module for calculating the "specialist-specialist average degree of correspondence" and a module for calculating the "specialist-AI medical device average degree of correspondence". Alternatively, all or some of functions of the statistical value derivation unit 130 may be included and implemented in the correspondence degree calculation unit 120. Alternatively, all or some of functions of the statistical value derivation unit 130 may be included and implemented in the comparison test unit 140 described below.

A clinical effectiveness evaluation system according to another embodiment of the present disclosure may not have a statistical value derivation unit 130. In this case, the comparison test unit 140, which will be described later, uses values of degrees of correspondence calculated in the specialist-specialist correspondence degree calculation unit 121 and values of degrees of correspondence calculated in the specialist-AI medical device correspondence degree calculation unit 122 to perform the clinical effectiveness evaluation.

The comparison test unit 140 may perform the test on the AI medical device on the basis of the statistical value derived from the statistical value derivation unit 130. According to an embodiment of the present disclosure, when the predetermined statistical value is an average, the comparison test unit 140 receives the "specialist-specialist average degree of correspondence" and the "specialist-AI medical device average degree of correspondence" and performs the test on the AI medical device on the basis on the same. Specifically, it is possible to test whether there is a difference between "specialist-specialist average degree of correspondence" and "specialist-AI medical device average degree of correspondence". That is, the test may be performed in such a manner to demonstrate the fact that the "specialist-AI medical device average degree of correspondence" is based on the "specialist-specialist average degree of correspondence". For the purpose thereof, hypotheses of various types (Type A to Type C) may be used as follows.

First, when the "specialist-specialist average degree of correspondence" and the "specialist-AI medical device average degree of correspondence" are referred to as µ₀ and µ₁, respectively, a null hypothesis H₀ and an alternative hypothesis H₁ are established as follows. When the null hypothesis is not rejected, the clinical test may be determined to be successful (Type A). That is, the AI medical device may be evaluated to have clinical effectiveness.

### <Type A>

H₀: µ₀ ≤ µ₁
H₁: µ₀ > µ₁

In this case, since the hypothesis to be tested is called the null hypothesis, a caution is needed in considering the significance level and the test power. By specifying the significance probability together, it is possible to determine the extent to which data supports the hypothesis.

As another embodiment, the lowest value m₀ among ten degrees of correspondence κ_{0,1} to κ_{0,10} calculated by the specialist-specialist correspondence degree calculation unit 121 is taken as a reference, and the null hypothesis H0 and the hypothesis H1 are established as follows. When the null hypothesis is rejected, the clinical test may be determined to be successful (Type B).

### <Type B>

H₀: m₀ ≥ µ₁
H₁: m₀ < µ₁

As a modified example of Type A, the null hypothesis H0 and the alternative hypothesis H1 for a specific value C are established as shown below. When the null hypothesis is rejected, the clinic test may be determined to be successful (Type C) .

### <Type C>

H₀: µ₀ - C ≥ µ₁
H₁: µ₀ - C < µ₁

As described above, according to the hypothesis used by the comparison test unit 140, the average or minimum value may be used as the statistical values of degrees of correspondence. However, the present disclosure is not limited thereto, and the statistical value derivation unit 130 may derive various statistic values, such as a weighted average, a cutting average, a maximum value, a median value, a fractile, a mode (most frequently occurring value), a variance, and a standard deviation of the degrees of correspondence, a standard error of the statistics, and the like, in addition to the average and the minimum value. The comparison test unit 140 can use the derived statistic values. Herein, the degree of correspondence may be the degree of correspondence between a specialist and a specialist, the degree of correspondence between a specialist and an AI medical device, or the degree of correspondence of both.

As described above, the comparison test unit 140 receives the statistical value as an input according to the hypothesis to be used, or directly receives κ_{0,1} to κ_{0,10} and/or κ_{1,1} to κ_{1,5} and then derives the statistic values required to test the hypothesis therefrom. The comparison test unit 140 receives the statistical value and then derives the statistical value using the statistical value derivation unit 130, for example.

According to the clinical effectiveness evaluation system 100 of the present disclosure, the opinions of two or more specialists are collected and used in the same clinical test environment, so that a more accurate and reliable reference may be obtained. In addition, clinical effectiveness evaluation may be performed even when no previous studies exist.

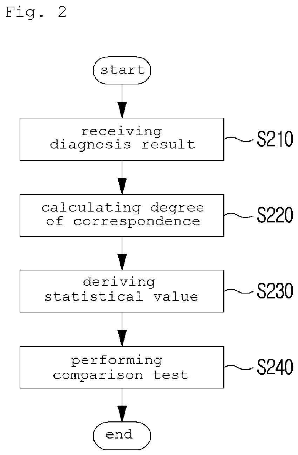

FIG. 2 is a diagram illustrating a method for clinical effectiveness evaluation of an artificial intelligence based medical device according to the present disclosure.

Each of the plurality of diagnostic subjects included in the diagnostic subject group may generate a diagnosis result for the same clinical data. The diagnostic subjects may include two or more specialists and an AI medical device, which is the subject of clinical effectiveness evaluation. For example, five specialists, "specialist A" to "specialist E" may generate a "diagnosis result A" to a "diagnosis result E", respectively, and an AI medical device may generate an "AI diagnosis result".

According to the clinical effectiveness evaluation method of the present disclosure, in step S210, the generated diagnosis results may be received. The operation of step S210 may be performed in the diagnosis result receiving unit 110 described with reference to FIG. 1.

In step S220, the degree of correspondence between the diagnosis results may be calculated on the basis of the received diagnosis results. The degree of correspondence between the diagnosis results may include the degree of correspondence between the diagnosis results of the two specialists and the degree of correspondence between the diagnosis results of the specialist and the AI medical device. As described above, when there are five specialists in the diagnosis subject group, a total of 10 degrees of correspondences may be obtained as the degree of correspondence between the diagnosis results of two specialists. In addition, when there are five specialists in the diagnostic subject group, a total of five degrees of correspondence may be obtained as the degree of correspondence between the diagnosis results of one specialist and the AI medical device. The degree of correspondence may be represented by a kappa statistic. The operation of step S220 may be performed in the correspondence degree calculation unit 120 described with reference to FIG. 1.

In step S230, the statistical value may be derived to perform the comparison test. For example, in order to perform the comparison test, when an average of the degrees of correspondence is used, an average of the degrees of correspondence calculated in step S220 may be derived in step S230. As described above, various statistical values such as a minimum value, a weighted average, a cutting average, a maximum value, a median value, a fractile, a mode, a variance, a standard deviation, and a standard error of the statistic may be used, in addition to the average of the degree of correspondence. In step S230, a predetermined statistical value selected therefrom may be derived. The operation of step S230 may be performed in the statistical value derivation unit 130 described with reference to FIG. 1.

In step S240, the comparison test may be performed using the derived statistical values. To perform the comparison test, at least one of the various types of hypotheses described with reference to FIG. 1 may be used. As a result of performing the comparison test, the effectiveness evaluation on whether the AI medical device has clinical effectiveness may be output. The operation of step S240 may be performed in the comparison test unit 140 described with reference to FIG. 1.

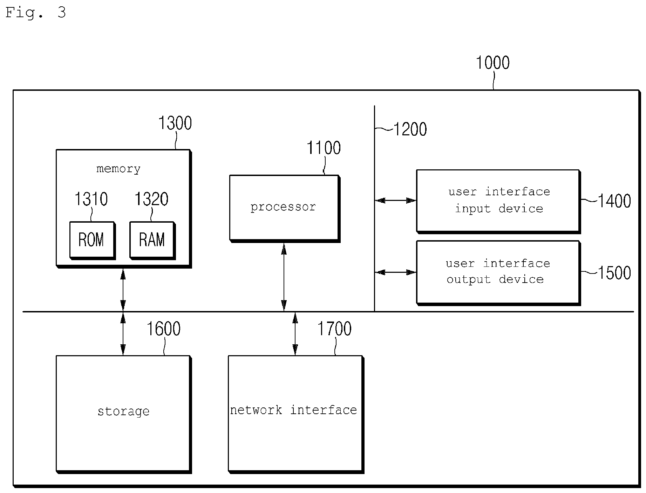

FIG. 3 is a block diagram illustrating a computing system that performs a method for clinical effectiveness evaluation of an artificial intelligence based medical device according to an embodiment of the present disclosure.

Referring to FIG. 3, a computing system 1000 includes at least one processor 1100 connected via a bus 1200, a memory 1300, a user interface input device 1400, a user interface output device 1500, a storage 1600, and a network interface 1700.

The processor 1100 may be a semiconductor device that performs processing for instructions stored in a central processing unit (CPU) or memory 1300 and/or a storage 1600. The memory 1300 and storage 1600 may include various types of volatile or non-volatile storage media. For example, the memory 1300 may include a Read Only Memory (ROM) and a Random Access Memory (RAM).

Thus, the steps of the method or algorithm described in connection with the embodiments disclosed herein may be embodied directly in hardware, a software module, or a combination of both, which are executed by the processor 1100. The software module may reside in a storage medium (i.e., memory 1300 and/or storage 1600) such as a RAM memory, a flash memory, a ROM memory, an EPROM memory, an EEPROM memory, a register, a hard disk, a removable disk, and CD-ROM. An exemplary storage medium is coupled to the processor 1100, which may read information from, and write information to, the storage medium. Alternatively, the storage medium may be integral with the processor 1100. The processor and the storage medium may reside within an application specific integrated circuit (ASIC). The ASIC may reside within the user terminal. Alternatively, the processor and the storage medium may reside as discrete components in a user terminal.

Although the exemplary methods of this disclosure are represented by a series of acts for clarity of explanation, they are not intended to limit the order in which the steps are performed, and if necessary, each step may be performed simultaneously or in a different order. In order to implement the method according to the present disclosure, the illustrative steps may additionally include other steps, include the remaining steps except for some steps, or may include additional steps other than some steps.

The various embodiments of the disclosure are not intended to be all-inclusive and are intended to illustrate representative aspects of the disclosure, and the features described in the various embodiments may be applied independently or in a combination of two or more.

In addition, various embodiments of the present disclosure may be implemented by hardware, firmware, software, or a combination of both. In the case of hardware implementation, it may be implemented by one or more application specific integrated circuits (ASICs), digital signal processors (DSPs), digital signal processing devices (DSPDs), programmable logic devices (PLDs), field programmable gate arrays (FPGAs), a general processor, a controller, a microcontroller, a microprocessor, and the like.

The scope of the present disclosure includes software or machine-executable instructions (e.g., operating system, applications, firmware, program, etc) that allow operations according to the various embodiments to be executable in device or computer, and a non-transitory computer-readable medium that is executable in the device or computer in which such software or instructions are stored.

## Claims

1. A system for clinical effectiveness evaluation of an artificial intelligence based medical device, the system comprising:
a diagnosis result receiving unit receiving diagnosis results for arbitrary clinical data from a plurality of specialists and the artificial intelligence based medical device (AI medical device);
a correspondence degree calculation unit calculating a degree of correspondence between the received diagnosis results;
a statistical value derivation unit deriving a predetermined statistical value on the basis of the calculated degree of correspondence; and
a comparison test unit performing a comparison test on the artificial intelligence based medical device using the derived statistical value.

2. The system of claim 1, wherein the correspondence degree calculation unit includes:
a specialist-specialist correspondence degree calculation unit; and
a specialist-AI medical device correspondence degree calculation unit,
in which the specialist-specialist correspondence degree calculation unit calculates a specialist-specialist degree of correspondence, which is a degree of correspondence between diagnosis results of two specialists, and
the specialist-AI medical device correspondence degree calculation unit calculates a specialist-AI medical device degree of correspondence, which is a degree of correspondence between a diagnosis result of one specialist and a diagnosis result of the AI-based medical device.

3. The system of claim 1, wherein the degree of correspondence is expressed as Cohen's kappa statistics.

4. The system of claim 1, wherein the predetermined statistical value derived from the statistical value derivation unit includes at least one of an average, a weighted average, a cutting average, a minimum value, a maximum value, an intermediate value, a fractile, a mode, a variance, and a standard deviation of the degrees of correspondence calculated in the correspondence degree calculation unit, and a standard error of the statistic.

5. The system of claim 2, wherein the statistical value derivation unit derives a specialist-specialist average degree of correspondence, which is an average of the specialist-specialist degrees of correspondence and a specialist-AI medical device average degree of correspondence, which is an average of the specialist-AI medical device degrees of correspondence, and
the comparison test unit performs the comparison test on the artificial intelligence based medical device by comparing the specialist-specialist average degree of correspondence with the specialist-AI medical device average degree of correspondence.

6. The system of claim 5, wherein the comparison test unit establishes a null hypothesis H₀ that the specialist-specialist average degree of correspondence is less than or equal to the specialist-AI medical device average degree of correspondence and an alternative hypothesis H₁ that the specialist-specialist average degree of correspondence is greater than the specialist-AI medical device average degree of correspondence, and
in which when the null hypothesis H0 is not rejected, the artificial intelligence based medical device is determined to have a clinical effectiveness.

7. The system of claim 2, wherein the statistical value derivation unit derives a minimum value of the specialist-specialist degrees of correspondence and a specialist-AI medical device average degree of correspondence, which is an average of the specialist-AI medical device degrees of correspondence, and
the comparison test unit performs the comparison test on the AI-based medical device by comparing the minimum value of the specialist-specialist degree of correspondence and the specialist-AI medical device average degree of correspondence.

8. The system of claim 7, wherein the comparison test unit establishes a null hypothesis H₀ that the minimum value of the specialist-specialist degrees of correspondence is greater than or equal to the specialist-AI medical device average degree of correspondence and an alternative hypothesis H₁ that the minimum value of the specialist-specialist degrees of correspondence is less than the specialist-AI medical device average degree of correspondence,
in which when the null hypothesis H₀ is rejected, it is determined that the artificial intelligence based medical device has a clinical effectiveness.

9. The system of claim 2, wherein the statistical value derivation unit derives the specialist-specialist average degree of correspondence, which is an average of the specialist-specialist degrees of correspondence, and the specialist-AI medical device average degree of correspondence, which is an average of the specialist-AI medical device degrees of correspondence, and
the comparison test unit performs the comparison test on the artificial intelligence based medical device by comparing the specialist-AI medical device average degree of correspondence with a modified specialist-specialist average degree of correspondence obtained by adding or subtracting a predetermined value to or from the specialist-AI medical device average degree of correspondence.

10. The system of claim 9, wherein the comparison test unit establishes a null hypothesis H₀ that the modified specialist-specialist average degree of correspondence is greater than or equal to the specialist-AI medical device average degree of correspondence and an alternative hypothesis H₁ that the modified specialist-specialist average degree of correspondence is less than the specialist-AI medical device average degree of correspondence,
in which when the null hypothesis H₀ is not rejected, it is determined that the artificial intelligence based medical device has a clinical effectiveness.

11. A method for clinical effectiveness evaluation of an artificial intelligence based medical device, the method comprising:
receiving unit receiving diagnosis results for arbitrary clinical data from a plurality of specialists and the artificial intelligence based medical device ("AI medical device");
calculating a degree of correspondence between the received diagnosis results;
deriving a predetermined statistical value on the basis of the calculated degree of correspondence; and
performing a comparison test on the artificial intelligence based medical device using the derived statistical value.

12. The method of claim 11, wherein the calculating of the degree of correspondence includes:
calculating a specialist-specialist correspondence degree; and
calculating a specialist-AI medical device correspondence degree,
in which the specialist-specialist correspondence degree is a degree of correspondence between diagnosis results of two specialists, and
the specialist-AI medical device correspondence degree is a degree of correspondence between a diagnosis result of one specialist and a diagnosis result of the AI-based medical device.

13. The method of claim 11, wherein the degree of correspondence is expressed as Cohen's kappa statistics.

14. The method of claim 11, wherein the predetermined statistical value derived from the statistical value derivation unit includes at least one of an average, a weighted average, a cutting average, a minimum value, a maximum value, an intermediate value, a fractile, a mode, a variance, and a standard deviation of the degrees of correspondence calculated in the correspondence degree calculation unit, and a standard error of the statistic.

15. The method of claim 12, wherein the deriving of the predetermined statistical value includes deriving a specialist-specialist average degree of correspondence, which is an average of the specialist-specialist degrees of correspondence and a specialist-AI medical device average degree of correspondence, which is an average of the specialist-AI medical device degrees of correspondence, and
the performing of the comparison test includes performing the comparison test on the artificial intelligence based medical device by comparing the specialist-specialist average degree of correspondence with the specialist-AI medical device average degree of correspondence.

16. The method of claim 15, wherein the performing of the comparison test includes establishing a null hypothesis H₀ that the specialist-specialist average degree of correspondence is less than or equal to the specialist-AI medical device average degree of correspondence and an alternative hypothesis H₁ that the specialist-specialist average degree of correspondence is greater than the specialist-AI medical device average degree of correspondence,
in which when the null hypothesis H0 is not rejected, the artificial intelligence based medical device is determined to have a clinical effectiveness.

17. The method of claim 12, wherein the deriving of the predetermined statistical value includes deriving a minimum value of the specialist-specialist degrees of correspondence and a specialist-AI medical device average degree of correspondence, which is an average of the specialist-AI medical device degrees of correspondence, and
the performing of the comparison test includes performing the comparison test on the AI-based medical device by comparing the minimum value of the specialist-specialist degree of correspondence and the specialist-AI medical device average degree of correspondence.

18. The method of claim 17, wherein the performing of the comparison test includes establishing a null hypothesis H₀ that the minimum value of the specialist-specialist degrees of correspondence is greater than or equal to the specialist-AI medical device average degree of correspondence and an alternative hypothesis H₁ that the minimum value of the specialist-specialist degrees of correspondence is less than the specialist-AI medical device average degree of correspondence,
in which when the null hypothesis H₀ is rejected, it is determined that the artificial intelligence based medical device has a clinical effectiveness.

19. The method of claim 12, wherein the deriving of the predetermined statistical value includes deriving the specialist-specialist average degree of correspondence, which is an average of the specialist-specialist degrees of correspondence, and the specialist-AI medical device average degree of correspondence, which is an average of the specialist-AI medical device degrees of correspondence, and
the performing of the comparison test includes performing the comparison test on the artificial intelligence based medical device by comparing the specialist-AI medical device average degree of correspondence with a modified specialist-specialist average degree of correspondence obtained by adding or subtracting a predetermined value to or from the specialist-AI medical device average degree of correspondence.

20. The method of claim 19, wherein the performing of the comparison test includes establishing a null hypothesis H₀ that the modified specialist-specialist average degree of correspondence is greater than or equal to the specialist-AI medical device average degree of correspondence and an alternative hypothesis H₁ that the modified specialist-specialist average degree of correspondence is less than the specialist-AI medical device average degree of correspondence,
in which when the null hypothesis H₀ is not rejected, it is determined that the artificial intelligence based medical device has a clinical effectiveness.
